# DEMANDE DE BREVET EUROPEEN

(11) **EP 2 873 382 A1**
(43) Date de publication de la demande: **20.05.2015**
(21) Numéro de dépôt: 14192057.9
(22) Date de dépôt: 06.11.2014
(51) Int. Cl.: A61B 17/42, A61B 17/34, A61B 17/00, A61F 2/08, A61F 2/00

(54) **Trocart à usage unique permettant la mise en place d'une proyhèse de suspension utérine pour le traitement des prolapsus utérins**

(30) Priorité: 14.11.2013 FR 1361111
(71) Demandeur: Cornier, Edgard, 75116 Paris (FR)
(72) Inventeur: Cornier, Edgard, 75116 Paris (FR)
(74) Mandataire: Cabinet HERRBURGER

(57) **Abrégé**

Trocart à usage unique permettant la mise en place d'une prothèse de suspension utérine (4) pour le traitement des prolapsus utérins, caractérisé en ce qu'il comporte d'une part trois pièces réalisées en un matériau stérilisable biocompatible moulé, à savoir :
- un corps de trocart (1) tubulaire de forme courbe réalisé en un matériau rigide constituant une gaine de guidage
- une poignée (2) susceptible d'être fixée à l'extrémité proximale (10) du corps de trocart (1), et
- un embout (3) constituant un organe de guidage susceptible d'être fixé à l'extrémité distale (11) du corps de trocart (1), et

d'autre part une bandelette en un tissu en un matériau biocompatible non résorbable constituant la prothèse (4) fixée à la poignée (2) et à l'embout (3) et traversant le corps de trocart (1), cette bandelette étant destinée à être sectionnée à ses deux extrémité pour permettre sa fixation par suture au col de l'utérus et aux aponévroses de la paroi abdominale.

## Description

La présente invention concerne un trocart à usage unique permettant la mise en place d'une prothèse de suspension utérine pour le traitement des prolapsus utérins.

Quel que soit son poids au cours de la vie génitale ou de la grossesse, l'utérus ne peut normalement pas tomber dans le vagin et est constamment maintenu dans une position haute par un ensemble de fibres conjonctives situées en arrière du péritoine et qui assurent le positionnement de l'utérus dans l'enceinte manométrique de l'abdomen.

Ces fibres conjonctives forment deux faisceaux ligamentaires à savoir un faisceau de ligaments allant du pubis au sacrum dits ligaments sacraux génitaux pubiens et un faisceau de ligaments obliques dirigés d'arrière en avant dits tente de l'hypogastrique.

Du fait de la géométrie particulière de la cavité péritonéale dans la zone de sa partie inférieure ou col, l'utérus est situé dans sa position haute normale au-dessus d'une pince de pression définie par la partie postérieure du péritoine (cul-de-sac de Douglas) et la partie antérieure du péritoine (cul-de-sac vésico-utérin) située entre l'utérus et la vessie.

En présence d'efforts abdominaux, ces deux culs-de-sac se referment sur l'entonnoir conique du col de l'utérus, de sorte que ce dernier soit refoulé vers le haut et ne puisse pas tomber dans le vagin.

Il est cependant relativement fréquent que les ligaments qui soutiennent l'utérus en particulier les fibres conjonctives latérales de la tente de l'hypogastrique se distendent et s'altèrent avec les grossesses ou l'âge ; une telle altération peut entraîner une descente de l'utérus hors de l'enceinte manométrique de l'abdomen ; il est alors expulsé de l'abdomen dans le vagin à chaque pression.

Le prolapsus utérin provoque une gêne fonctionnelle pouvant ne se manifester que lors d'efforts intenses ou être permanente, et donc avoir un caractère fortement invalidant.

Les spécialistes proposent divers moyens de traitement des prolapsus utérins consistant notamment à fixer l'utérus à la colonne vertébrale, à fermer le cul-de-sac de Douglas, voire à enlever l'utérus.

Aucun de ces moyens n'est toutefois de nature à donner entière satisfaction comme l'a montré la multiplicité des techniques prônées depuis longtemps.

Pour remédier à ces inconvénients, on a déjà eu l'idée selon le document FR 2 874 330 A de mettre en place entre le péritoine et la paroi abdominale une prothèse constituée par une bandelette en un tissu, notamment en un treillis en un matériau synthétique biocompatible non résorbable et de suturer cette bandelette par des fils non résorbables d'une part au col de l'utérus par sa partie inférieure et d'autre part aux aponévroses de la paroi abdominale par sa partie supérieure en remplacement des fibres conjonctives défaillantes.

Une telle prothèse donne globalement satisfaction dans la mesure où elle permet de corriger de façon satisfaisante, le prolapsus utérin en replaçant l'utérus au-dessus de la pince de pression du cul-de-sac de Douglas qu'il importe de respecter, et donc dans l'enceinte manométrique de l'abdomen.

Pour la bonne réussite de la correction, il est essentiel que les prothèses ne soient pas fixées sous forte tension ; le but à obtenir est en effet uniquement le maintien de l'utérus dans une position proche de celle où le placent les fragiles ligaments obliques latéraux et les ligaments sacraux génitaux pubiens.

Le document FR 2 874 330 A propose à cet effet un dispositif permettant la mise en place d'une telle prothèse de suspension utérine qui est essentiellement constitué par un corps de trocart tubulaire guide de forme hélicoïdale réalisé en un matériau rigide et par un fil de liaison en matière synthétique susceptible de coulisser à la partie interne de ce corps de trocart.

Ce fil de liaison fait saillie à l'extérieur du corps de trocart par une première de ses extrémités ou extrémité proximale et par sa seconde extrémité ou extrémité distale au niveau de laquelle il est muni d'un embout percé d'un chas permettant de glisser la prothèse.

Le positionnement de cette prothèse nécessite la mise en place préalable du corps de trocart qui doit trouver un passage entre la paroi abdominale et le péritoine jusqu'à ce que l'extrémité distale du corps de trocart équipé de l'embout parvienne dans le vagin au niveau duquel le praticien peut introduire la prothèse dans son chas.

Le praticien peut ensuite retirer le corps de trocart du bas vers le haut de sorte qu'il entraine la prothèse avec lui, avant de fixer cette prothèse d'une part au col de l'utérus par sa partie inférieure et d'autre part aux aponévroses abdominales par sa partie supérieure.

La mise en place d'un tel dispositif s'avère relativement longue et peu commode pour le praticien du fait de la présence du fil de liaison et de la nécessité de glisser manuellement la prothèse dans l'embout équipant ce fil de liaison à son extrémité distale, ce dans le vagin de la patiente.

La présente invention a pour objet de remédier à cet inconvénient en proposant un trocart à usage unique permettant la mise en place d'une prothèse de suspension utérine pour le traitement des prolapsus utérins du type susmentionné ne nécessitant pas l'utilisation d'un fil de liaison traversant le corps de trocart à sa partie interne.

Selon l'invention, un tel trocart à usage unique comporte trois pièces réalisées en un matériau stérilisable biocompatible moulé à savoir un corps de trocart tubulaire de forme courbe réalisé en un matériau rigide constituant une gaine de guidage, une poignée susceptible d'être fixée à l'extrémité proximale du corps de trocart en particulier par vissage ou encliquetage et un embout de préférence en mousse arrondi en ogive constituant un organe de guidage susceptible d'être fixé à l'extrémité distale du corps de trocart là encore en particulier par vissage ou encliquetage.

De façon connue en elle-même, le corps de trocart peut être éventuellement muni au voisinage de son extrémité proximale d'un raccord latéral de type luer susceptible d'être branché sur des organes de perfusion et au voisinage de son extrémité distale d'un ensemble de perforations de type crépine permettant l'évacuation vers l'extérieur d'un liquide de perfusion en particulier de sérum physiologique.

Selon l'invention, le trocart comporte également une bandelette en un tissu notamment en un treillis en un matériau biocompatible non résorbable constituant la prothèse qui est fixée à la poignée et à l'embout et traverse le corps de trocart.

Cette bandelette, qui remplace en fait le fil de liaison équipant le dispositif antérieur décrit dans le document FR 2 8754 330 A est destinée à être sectionnée à ses deux extrémités après la mise en place du corps de trocart pour permettre sa fixation par suture au col de l'utérus et aux aponévroses de la paroi abdominale.

Cette fixation se trouve ainsi notablement facilitée.

Plus précisément, la fixation de la prothèse nécessite la mise en place préalable du corps de trocart qui doit trouver un passage entre la paroi abdominale et le péritoine.

Cette opération qui a déjà été décrite exige la perforation de l'abdomen de la patiente par une incision cutanée pratiquée à environ 3 cm au-dessus et en dedans des épines iliaques puis l'introduction de l'extrémité distale du corps de trocart équipée de l'embout par l'orifice ainsi créé.

Préalablement à l'introduction du corps de trocart, le chirurgien doit préparer par les voies naturelles une incision péri cervicale et faiblement décoller la partie basse de l'accolement entre la vessie et l'isthme utérin.

Le praticien doit ensuite déplacer l'ensemble corps de trocart / prothèse entre le péritoine et la paroi abdominale en le faisant progresser vers le bas et vers l'avant de l'abdomen, ce jusqu'au niveau du col de l'utérus en passant en arrière de la vessie.

Au cours de la progression de cet ensemble dans la cavité péritonéale le raccord latéral de type luer du corps de trocart peut éventuellement, dans la mesure où il est présent, être constamment branché sur une poche de perfusion renfermant du sérum physiologique.

Selon cette variante, du sérum physiologique est constamment évacué vers l'extérieur au voisinage immédiat de l'extrémité distale alors munie d'une crépine.

Ce sérum a pour fonction de permettre d'ouvrir les espaces en décollant le péritoine de la paroi abdominale ainsi que des vaisseaux iliaques de l'urètre et de la vessie de façon à permettre d'accéder en arrière de cette dernière, au col de l'utérus à maintenir.

Le passage du corps de trocart peut être réalisé sous contrôle visuel (coelioscopie) vers le ligament large, et la face antérieure de l'utérus puis tactile derrière la vessie et le col de l'utérus à la fin du trajet.

Le corps de trocart peut aussi être guidé sous contrôle échographique grâce à une perfusion de sérum physiologique sur la partie haute du trajet puis derrière la vessie gonflée par environ 300 cc de sérum physiologique sur la seconde partie du trajet.

A la fin du trajet, le corps de trocart est guidé par voie tactile au niveau de la face antérieure de l'isthme utérin.

En fin de progression l'extrémité distale équipée de l'embout du corps de trocart est proche du doigt du chirurgien introduit dans le vagin qui peut alors guider son passage et maintenir l'embout sur lequel est fixée la prothèse.

Le praticien peut ensuite séparer l'embout du corps de trocart et sectionner la prothèse au niveau de son extrémité proximale avant de retirer le corps de trocart du bas vers le haut.

La prothèse se trouve ainsi positionnée et il ne reste ensuite plus au praticien qu'à sectionner cette prothèse au niveau de son extrémité distale et à la fixer d'une part au col de l'utérus par sa partie inférieure et d'autre part aux aponévroses abdominales par sa partie supérieure.

Le diamètre et la longueur du corps de trocart du trocart conforme à l'invention dépendent largement de la taille et de la corpulence des patientes ; toutefois le corps de trocart a en règle générale une longueur de l'ordre de 25 à 30 cm et un diamètre de l'ordre de 5 mm.

Pour faciliter la progression du corps de trocart au cours de son trajet sous péritonéal, celui-ci comporte de préférence, en allant de son extrémité proximale à son extrémité distale une première partie rectiligne ou légèrement concave vers l'avant dans le plan de l'axe de la poignée puis une seconde partie recourbée en dedans selon environ un tiers de circonférence dont le cadre mesure environ 10 centimètres.

Il peut ainsi exister plusieurs tailles de trocart, adaptées à la corpulence des opérées, qui retiendront les caractéristiques de courbure du trocart.

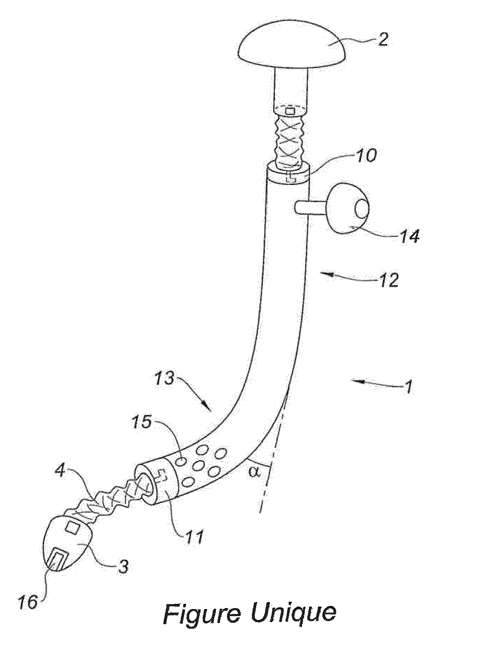

Les caractéristiques du trocart qui fait l'objet de l'invention seront décrites plus en détail en se référant au dessin non limitatif annexé qui est une vue en perspective schématique de ce trocart.

Selon la figure, le trocart comporte un corps de trocart tubulaire 1 réalisé en un matériau rigide, une poignée 2 susceptible d'être fixée à l'extrémité proximale 10 du corps de trocart 1 par vissage ou encliquetage ainsi qu'un embout mousse arrondi en ogive 3 constituant un organe de guidage susceptible d'être fixé à l'extrémité distale 11 du corps de trocart 1, là encore par vissage ou encliquetage.

Ces trois pièces 1, 2 et 3 sont réalisées en un matériau stérilisable biocompatible moulé.

Une prothèse 4 constituée par une bandelette en un treillis en un matériau biocompatible non résorbable en particulier en un poly(éthylène téréphtalate) tel que le polymère commercialisé sous la dénomination Mersilène® est fixée à la poignée 2 et à l'embout 3 et traverse le corps de trocart 1 qui la protège.

Ce corps de trocart 1 a une longueur de l'ordre de 25 à 30 cm et un diamètre de l'ordre de 5 mm et comporte en allant de son extrémité proximale 10 à son extrémité distale 11 une première partie 12 rectiligne puis une seconde partie 13 recourbée en dedans.

Selon l'exemple de réalisation représenté sur la figure, le corps de trocart 1 est par ailleurs muni au voisinage de son extrémité proximale 10 d'un raccord latéral de type luer 14 susceptible d'être branché sur des organes de perfusion non représentés, et au voisinage de son extrémité distale 11 d'un ensemble de perforations de type crépine 15 permettant l'évacuation vers l'extérieur d'un liquide de perfusion, en particulier de sérum physiologique.

L'embout 3 comporte quant à lui une cavité 16 de nature à faciliter sa préhension.

### NOMENCLATURE

- 1.: Corps de trocart
- 2.: Poignée
- 3.: Embout en mousse
- 4.: Prothèse
- 10.: Extrémité proximale du corps de trocart 1
- 11.: Extrémité distale du corps de trocart 1
- 12.: Première partie du corps de trocart 1
- 13.: Seconde partie du corps de trocart 1
- 14.: Raccord latéral de type luer
- 15.: Ensemble de perforations de type crépine
- 16.: Cavité

## Revendications

1. Trocart à usage unique permettant la mise en place d'une prothèse de suspension utérine (4) pour le traitement des prolapsus utérins, cette prothèse (4) étant destinée à être mise en place entre le péritoine et la paroi abdominale et à être suturée par des fils non résorbables d'une part au col de l'utérus par sa partie inférieure et d'autre part aux aponévroses de la paroi abdominale par sa partie supérieure en remplacement des fibres conjonctives défaillantes,
**caractérisé en ce qu'**
il comporte :
- d'une part trois pièces réalisées en un matériau stérilisable biocompatible moulé, à savoir :
- un corps de trocart (1) tubulaire de forme courbe réalisé en un matériau rigide constituant une gaine de guidage,
- une poignée (2) susceptible d'être fixée à l'extrémité proximale (10) du corps de trocart (1), en particulier par vissage ou encliquetage, et
- un embout (3) arrondi en ogive constituant un organe de guidage susceptible d'être fixé à l'extrémité distale (11) du corps de trocart (1), en particulier par vissage ou encliquetage, et
- d'autre part une bandelette en un tissu notamment en un treillis en un matériau biocompatible non résorbable constituant la prothèse (4) fixée à la poignée (2) et à l'embout (3) et traversant le corps de trocart (1), cette bandelette étant destinée à être sectionnée à ses deux extrémité pour permettre sa fixation par suture au col de l'utérus et aux aponévroses de la paroi abdominale.

2. Trocart conforme à la revendication 1,
**caractérisé en ce que**
le corps de trocart (1) a une longueur de l'ordre de 25 à 30 cm et un diamètre de l'ordre de 5 mm.

3. Trocart conforme à l'une quelconque des revendications 1 et 2, **caractérisé en ce que**
le corps de trocart (1) comporte en allant de son extrémité proximale (10) à son extrémité distale (11) une première partie (12) rectiligne puis une seconde partie (13) recourbée en dedans.

4. Trocart conforme à l'une quelconque des revendications 1 à 3, **caractérisé en ce que**
l'embout (3) comporte une cavité (16) de nature à faciliter sa préhension.
